# EUROPÄISCHE PATENTSCHRIFT

(11) **EP 2 235 514 B1**
(45) Veröffentlichungstag und Bekanntmachung des Hinweises auf die Patenterteilung: **10.01.2018**
(21) Anmeldenummer: 09701586.1
(22) Anmeldetag: 16.01.2009
(51) Int. Cl.: G01N 27/404

(54) **GASSENSOR MIT MIKROPORÖSER ELEKTROLYTSCHICHT**
GAS SENSOR WITH MICROPOROUS ELECTROLYTE LAYER
CAPTEUR DE GAZ DOTÉ D'UNE COUCHE D'ÉLECTROLYTE MICROPOREUSE

(30) Priorität: 18.01.2008 EP 08000943
(43) Veröffentlichungstag der Anmeldung: 06.10.2010
(73) Patentinhaber: F. Hoffmann-La Roche AG, 4070 Basel (CH); Roche Diagnostics GmbH, 68305 Mannheim (DE)
(72) Erfinder: OFFENBACHER, Helmut, A-8020 Graz (AT); STEINER, Gregor, 23558 Lübeck (DE); MURESANU, Claudia-Gemma, 8054 Graz (AT)
(74) Vertreter: Weickmann & Weickmann PartmbB
(86) Internationale Anmeldenummer: PCT/EP2009/000273
(87) Internationale Veröffentlichungsnummer: WO 2009/090094

(56) Entgegenhaltungen:
- EP-A- 0 097 554
- EP-A- 0 299 780
- DE-A1- 19 859 198
- GB-A- 2 308 193
- US-A1- 2004 033 414
- US-A1- 2006 021 873
- US-A1- 2006 293 169

## Beschreibung

Die vorliegende Erfindung betrifft elektrochemische Sensoren zur Bestimmung von in einem wässrigen Messmedium gelösten gasförmigen Analyten, ein Verfahren zu deren Herstellung, sowie ein Verfahren zur Bestimmung von in einem wässrigen Messmedium gelösten gasförmigen Analyten unter Verwendung der elektrochemischen Sensoren. Die Erfindung betrifft insbesondere den Reaktionsraum der elektrochemischen Sensoren, welcher vom wässrigen Messmedium durch eine gaspermeable und ionenundurchlässige Deckschicht räumlich abgetrennt ist.

Messsysteme zur Analytik von Körperflüssigkeiten stellen wichtige Bestandteile klinisch relevanter Analyseverfahren dar. Hierbei steht insbesondere eine schnelle und präzise Messung von Analyten im Vordergrund, welche die Bestimmung sogenannter "Point-of-Care"-Parameter gestattet. Point-of-Care-Untersuchungen haben den Vorteil, dass die Ergebnisse bereits nach kurzer Zeit vorliegen, da zum einen der Transport der Proben zu einem spezialisierten Labor entfällt und zum anderen keine Rücksicht auf die zeitlichen Abläufe des Labors genommen werden muss. Point-of-Care-Untersuchungen werden vor allem auf Intensivstationen und in der Anästhesie, aber auch in Ambulanzen durchgeführt. Zu diesen so genannten Notfallparametern zählen unter anderem die Blutgaswerte (pCO₂, pO₂), der pH-Wert, Elektrolytwerte (z.B. Na⁺, K⁺, Ca²⁺, Cl⁻), sowie gewisse Metabolitenwerte.

Zur Durchführung derartiger Point-of-Care-Untersuchungen können beispielsweise Teststreifen oder auch medizinische Analysatoren mit Multi-Use-Sensoren verwendet werden, wodurch der manuelle Aufwand für die Durchführung auf ein Minimum reduziert wird. Messgeräte für einen Point-of-Care-Einsatz sind in der Regel nahezu vollständig automatisiert und erfordern von der Probenvorbereitung bis zum Testergebnis nur wenige und einfache Eingriffe des Benutzers. Sie können sowohl für eine einzige als auch für eine wiederholte Messung der zu bestimmenden Parameter ausgebildet sein.

Zur Messung gasförmiger Analyte, wie beispielsweise in Vollblut bzw. anderen wässrigen Medien gelöstem Sauerstoff oder Kohlendioxid, haben sich elektrochemische Sensoren als besonders geeignet erwiesen. Elektrochemische Sensoren gestatten die Messung eines Analyten mittels zweier oder mehrerer Elektroden, wobei mindestens eine der Elektroden die Arbeitselektrode darstellt, an welcher der zu bestimmende Analyt elektrochemisch verändert (z.B. oxidiert oder reduziert) wird.

Die Messung von Sauerstoff bzw. dessen Partialdruck (pO₂) in einem wässrigen Messmedium kann beispielsweise unter Verwendung amperometrischer Sensoren, umfassend mindestens eine Arbeitselektrode und mindestens eine Gegenelektrode, erfolgen. Bei Elektroden vom Clark-Typ trennt in der Regel eine gasdurchlässige und weitgehend ionen- und flüssigkeitsundurchlässige Membran den Probenraum räumlich vom Innenelektrolytraum ab. Der Innenelektrolytraum ist mit einer Elektrolytlösung befüllt, in welcher sich eine Arbeitselektrode und eine Gegenelektrode befinden.

Die Messung von Kohlendioxid bzw. dessen Partialdruck (pCO₂) in Flüssigkeiten oder Gasen kann beispielsweise unter Verwendung potentiometrischer Sensoren, umfassend mindestens eine Messelektrode und mindestens eine Referenzelektrode, vorgenommen werden. Bei Elektroden vom Severinghaus-Typ wird die Messung des CO₂-Partialdrucks auf eine pH-Messung zurückgeführt. Hierzu ist im Allgemeinen ein Reaktionsraum erforderlich, welcher vom wässrigen Messmedium durch eine gaspermeable und weitgehend ionenundurchlässige Membran räumlich abgetrennt ist. In diesem Reaktionsraum wird der pH-Wert gemessen, welcher durch den jeweiligen pCO₂-Wert der zu messenden Probe bestimmt wird. Bei vorgegebener Temperatur und Konzentration der Pufferlösung im Reaktionsraum (Innenelektrolyt) ist der pH-Wert des Reaktionsraumes ausschließlich vom CO₂-Partialdruck der Probe abhängig. Die Detektion des pH-Wertes kann auf unterschiedlichste Weise erfolgen, wie z. B. potentiometrisch über eine elektrochemische Messkette unter Verwendung ionenselektiver Glaselektroden oder ionensensitiver bzw. ionenselektiver Feldeffekttransistoren (ISFET), pH-sensitiver Festkörpersysteme (z. B. Edelmetall/Edelmetalloxid-Systeme), Redoxsysteme (Chinhydronelektrode), etc.

Ein wichtiges Kriterium bei der Bereitstellung elektrochemischer Sensoren ist deren Lebensdauer. Hierbei besteht das Bedürfnis, sowohl eine dauerhafte Lagerfähigkeit vor Inbetriebnahme des Sensors als auch eine hohe "in-use"-Lebensdauer zu erzielen. Um eine dauerhafte Lagerfähigkeit zu gewährleisten, sollten die in elektrochemischen Sensoren befindlichen Elektroden trocken, d. h. im Wesentlichen wasserfrei, gelagert und erst unmittelbar vor Inbetriebnahme mit dem flüssigen Innenelektrolyten in Kontakt gebracht werden. Um eine "in-use"-Lebensdauer von wenigstens 500 Messungen oder 3 bis 4 Wochen zu erreichen, müssen die verschiedenen Schichten des Sensors ferner kompatibel zueinander sein. Keinesfalls dürfen sie sich voneinander ablösen oder Risse ausbilden, wie beispielsweise durch Quellung.

Ein weiteres wichtiges Kriterium bei der Bereitstellung elektrochemischer Sensoren für Point-of-Care-Untersuchungen sind deren Maße. Zur Bestimmung von Notfallparametern stehen im Allgemeinen geringe Probenmengen (z. B. 100 µl oder weniger) zur Verfügung. Soll mit geringen Probenmengen eine Vielzahl von Parametern bestimmt werden, müssen die einzelnen Elektroden sowie deren Abstände zueinander möglichst klein sein.

GB 2 308 193 betrifft einen elektrochemischen Gassensor, welcher eine gaspermeable Messelektrode, eine Gegenelektrode sowie einen die Messelektrode und die Gegenelektrode verbindenden Elektrolyten umfasst, wobei die Messelektrode auf gegenüber liegenden Seiten des Elektrolyten eine Vielzahl an gasabsorbierenden Oberflächen aufweist. Die Elektroden sind auf einer bevorzugt aus PTFE bestehenden gaspermeablen Membran angeordnet, welche in Form eines Rahmens ausgestaltet sein kann, um dem Gassensor die nötige Stabilität und Festigkeit zu verleihen. Die Elektrolytschicht besteht ihrerseits bevorzugt aus einem Vliesstoff, welcher mit flüssigem Elektrolyten gesättigt ist, wobei anstelle von Vliesstoff auch Glas, Keramik oder Kunststoff, jeweils in poröser Form, als Elektrolytträger dienen können.

US 2006/0293169 A1 offenbart ein poröses Nanozeolithmaterial, ein Verfahren zu dessen Herstellung sowie Gassensoren, welche ein derartiges Material umfassen. Die Herstellung des Nanozeolithmaterials umfasst das Inkontaktbringen einer geeigneten Vorläuferverbindung (z.B. Tetraethylorthosilikat) mit einem strukturdirigierenden Mittel (z.B. Tetrapropylammoniumhydroxid), Wasser und Base in einem vorbestimmten Molverhältnis, Rühren bis zu einer gewünschten Viskosität, Altern bei einer gewünschten Temperatur und einem gewünschten Druck, Zerkleinern der synthetisierten Zeolithpartikel, Calcinieren der Zeolithpartikel zum Entfernen des strukturdirigierenden Mittels sowie Mischen des erhaltenen Zeolithmaterials mit einem Polymer, welches dem Zeolithmaterial für nachfolgendes (Elektro-)Spinnen die erforderliche Viskosität verleiht.

DE 198 59 198 beschreibt einen elektrochemischen Gassensor mit einem Elektrolyt aus poröser Keramik, welche mit Elektrolytflüssigkeit getränkt ist. Der Sensor weist eine Diffusionsmembran für ein nachzuweisendes Zielgas auf, wobei die Diffusionsschicht für Flüssigkeiten undurchlässig ist und den Elektrolyt gegenüber der Umgebung abschließt.

EP 0 805 973 B1 offenbart eine Vorrichtung sowie ein Verfahren zum Messen der Konzentration von Gasen in einer Probe. Als Vorrichtung dient ein elektrochemischer Sensor, welcher eine Arbeitselektrode, eine Gegen- bzw. Referenzelektrode, eine Elektrolytschicht und eine gaspermeable Membran umfasst, wobei die Elektrolytschicht aus einem fotogeformten proteinösen Gallert besteht. Um eine frühzeitige Kontamination der negativ polarisierten, aus Gold bestehenden Arbeitselektrode (Kathode) durch positiv geladene, von der aus Silber bestehenden Gegenelektrode (Anode) stammende Silberionen zu vermeiden, muss der Abstand der beiden durch eine Gallertschicht in elektrischem Kontakt stehenden Elektroden mindestens 1 mm betragen.

US 5,387,329 beschreibt einen planaren elektrochemischen Sauerstoffsensor, bei dem ein quellbares Polymer, dessen Quellrate unter dem Zweifachen seines Trockenvolumens liegt, als hygroskopischer Elektrolyt verwendet wird und hierbei eine hydrophile, für Wasser und Kationen permeable Elektrolytschicht ausbildet. Ein im Rahmen dieses Dokuments bevorzugtes quellbares Polymer ist Nafion®, ein sulfoniertes Tetrafluorethylenpolymer, dessen mit Lithium beladene Sulfonatgruppen dem Polymer ionomere Eigenschaften verleihen und einen Austausch von Lithiumionen gegen Silberionen bewirken. Dies hat den Effekt, dass bei einem amperometrischen Sauerstoffsensor mit Silber-Gegenelektrode die effektive Wanderungsgeschwindigkeit der Silberionen zur Arbeitselektrode erniedrigt wird.

Die in EP 0 805 973 B1 und US 5,387,329 verwendeten Elektrolytschichten sind mit Nachteilen behaftet. So ist beispielsweise die Herstellung sehr dünner Schichten (ca. 1 µm) quellfähiger Polymere (wie z.B. die in EP 0 805 973 B1 fotogeformten proteinösen Gallerte) sehr aufwendig.

Weiterhin führen die beim Betrieb eines Sauerstoffsensors freigesetzten Silberionen bei sehr dünnen Schichten und sehr kleinen Elektrolytvolumina schnell zu Störsignalen. Bei Verwendung dickerer, quellender Schichten (ca. 10-50 µm) in einem mehrschichtigen Aufbau wird andererseits die Bildung von Undichtigkeiten im Schichtaufbau erleichtert. Hierdurch bereitet es Schwierigkeiten, die angestrebte Langlebigkeit des Sensors zu erreichen. Ein bedeutender Nachteil dünner, wasserhaltiger Polymerschichten ist darüber hinaus, dass störende Silberionen nach Anlegen eines elektrischen Feldes zwischen den beiden Elektroden auf direktem Weg durch das Polymer wandern und so nach relativ kurzer Betriebsdauer zur Arbeitselektrode gelangen und diese kontaminieren.

Die der Erfindung zugrunde liegende Aufgabe bestand darin, einen elektrochemischen Sensor zur Bestimmung eines gasförmigen Analyten bereitzustellen, bei welchem die Nachteile des Standes der Technik zumindest teilweise beseitigt sind. Insbesondere sollte der Sensor dauerhaft gelagert werden können, ohne hierbei an Aktivität zu verlieren, und sollte darüber hinaus nach Inbetriebnahme eine hohe "in-use"-Lebensdauer aufweisen. Weiterhin sollte der Sensor einfach und kostengünstig hergestellt werden können.

Diese Aufgabe wurde erfindungsgemäß gelöst mittels eines elektrochemischen Sensors zur Bestimmung eines in einem wässrigen Messmedium gelösten gasförmigen Analyten, umfassend:
(a) eine Arbeitselektrode und eine Gegenelektrode,
(b) eine zwischen der Arbeitselektrode und der Gegenelektrode lokalisierte Elektrolytschicht,
(c) eine gaspermeable Deckschicht, welche zur räumlichen Abtrennung der Arbeitselektrode, der Gegenelektrode und der Elektrolytschicht vom wässrigen Messmedium dient,
dadurch gekennzeichnet,
dass die Elektrolytschicht mindestens ein partikelförmiges Material und mindestens ein Bindemittel umfasst, welche zusammen eine poröse, nicht-quellbare Gerüststruktur ausbilden, wobei die Poren der nicht-quellbaren Gerüststruktur zur Aufnahme eines flüssigen Elektrolyten vorgesehen sind oder diesen enthalten, und
(i) die Poren der nicht-quellbaren Gerüststruktur einen Durchmesser von etwa 500 nm bis etwa 5 µm, insbesondere von etwa 1 µm bis etwa 3 µm aufweisen, oder/und
(ii) die poröse, nicht-quellbare Gerüststruktur eine relative Flüssigkeitsaufnahme von etwa 20 Gew.-% bis etwa 50 Gew.-%, bevorzugt von etwa 26 Gew.-% bis etwa 44 Gew.-%, bezogen auf das Trockengewicht der porösen, nicht-quellbaren Gerüststruktur, bewirkt.

Der Ausdruck "Bestimmung eines in einem wässrigen Medium gelösten gasförmigen Analyten", wie er im Rahmen dieser Anmeldung verwendet wird, umfasst sowohl die Bestimmung des Vorhandenseins bzw. der Konzentration der gelösten Gaskomponente im wässrigen Medium als auch die Bestimmung des Gaspartialdrucks der gelösten Gaskomponente im wässrigen Medium.

Erfindungsgemäß befinden sich die Poren der nicht-quellbaren Gerüststruktur zwischen den Partikeln des mindestens einen partikelförmigen Materials. Im Vergleich zu einer reinen Polymerschicht bzw. Gallert- oder Gelschicht erhöht das auf diese Weise gebildete Porenlabyrinth die Wegstrecke, welche von den sich zwischen den Elektroden bewegenden Ionen zu durchlaufen ist, und erhöht somit die Lebensdauer des Sensors.

Partikelförmige Materialien, welche in den elektrochemischen Sensoren der vorliegenden Erfindung Anwendung finden, können anorganischer oder organischer Natur sein und umfassen insbesondere dichte anorganische Materialien oder dichte (weichmacherfreie) organische Polymere. Geeignete anorganische partikelförmige Materialien umfassen Controlled Porous Glass (CPG), Kieselgele, Silikate und Alumosilikate (auch Aluminosilikate genannt), welche vornehmlich aus der Gruppe bestehend aus Gerüstsilikaten und Schichtsilikaten ausgewählt sind. Organische Polymere, welche erfindungsgemäß als partikelförmiges Material eingesetzt werden können, umfassen beispielsweise Mikropartikel aus Polystyrol, Polyamid, Polyvinylchlorid, Polyvinylidenchlorid, Poly(meth)acrylat, Polyacrylnitril, sowie Copolymere hiervon, sind jedoch nicht auf diese beschränkt.

Als vorteilhaft hat sich im Rahmen der Erfindung die Verwendung eines natürlich vorkommenden oder synthetischen Alumosilikats, stärker bevorzugt eines synthetischen Alumosilikats, als partikelförmiges Material erwiesen. Der Begriff "synthetisches Alumosilikat", wie er im Rahmen der vorliegenden Erfindung verwendet wird, umfasst sowohl vollsynthetische Alumosilikate als auch Alumosilikate, welche durch künstliche Veränderung (z.B. chemisch) eines natürlich vorkommenden Alumosilikats erhalten werden. Beispiele für natürlich vorkommende oder synthetische Alumosilikate umfassen Feldspäte, Glimmer, Mullit, Sillimanit und Zeolithe, sind jedoch nicht auf diese beschränkt.

In einer bevorzugten Variante des erfindungsgemäßen elektrochemischen Sensors weist das partikelförmige Material Kanäle, insbesondere Kanäle mit einem Durchmesser zwischen etwa 0,1 nm bis etwa 10 nm, und optional lonentauschergruppen auf. In einer stärker bevorzugten Ausführungsform handelt es sich bei dem partikelförmigen Material um ein Zeolith, wie beispielsweise Faujasit, Chabasit, Mordenit oder Natrolith, in welchem Polyeder, Schichten oder Ketten aus eckenverknüpften [(Al,Si)O₄]-Tetraedern vorliegen, die ein von Kanälen durchzogenes, anionisches Raumnetzwerk ausbilden. Je nach Art des Zeoliths weisen die Kanäle einen Durchmesser von etwa 0,5 nm bis etwa 5,0 nm, bevorzugt von etwa 0,7 nm bis etwa 2,0 nm, auf und enthalten an ihren inneren und äußeren Oberflächen lonentauschergruppen, insbesondere Kationentauschergruppen. Die Verwendung von Zeolithen mit innerer Kanalstruktur und lonentauschergruppen hat sich insbesondere bei amperometrischen Elektroden vom Clark-Typ als besonders vorteilhaft erwiesen. In einer besonders bevorzugten Ausführungsform umfasst der erfindungsgemäße Sensor Faujasit, am stärksten bevorzugt Faujasit-Na, als partikelförmiges Material.

Infolge ihrer kanalhaltigen Ausgestaltung weisen Zeolithe eine große innere Oberfläche auf, welche mit einem flüssigen Elektrolyten in Kontakt treten kann. Da andererseits Ionen geeigneter Größe, welche in der Elektrolytflüssigkeit enthalten sind, durch die Kanäle des Zeolithen wandern können, kann die Menge an unerwünschten Ionen reduziert werden. So tritt bei elektrochemischen Sensoren, welche silberhaltige Gegenelektroden enthalten, insbesondere das Problem auf, dass mit Inbetriebnahme des Sensors Silberionen aus der Gegenelektrode freigesetzt werden, in Richtung der Arbeitselektrode wandern und sich unter Passivierung der Arbeitselektrode als elementares Silber auf dieser absetzen. Ähnliche Vorgänge laufen auch bei anderen als Elektrodenmaterialien eingesetzten Metallen wie beispielsweise Blei ab, welches bei Kontakt mit der Elektrolytflüssigkeit Bleiionen freisetzen kann, die ebenfalls als Störkationen wirken können. Durch Verwendung eines elektrochemischen Sensors gemäß der vorliegenden Erfindung, dessen Elektrolytschicht bevorzugt mindestens ein Zeolith umfasst, kann die effektive Wegstrecke zwischen Gegenelektrode und Arbeitselektrode vergrößert und damit die Gefahr einer raschen Passivierung der Arbeitselektrode verringert werden, was sich in einer verbesserten Lebensdauer des Sensors äußert.

Andererseits können Zeolithe aufgrund ihrer anionischen Gerüststruktur auch als Ionentauscher wirken. So können beispielsweise an der Gegenelektrode freigesetzte und zur Arbeitselektrode wandernde Silberionen von Zeolithen absorbiert und gegen geeignete, weniger kritische Kationen, wie z.B. Natriumionen, ausgetauscht werden, wodurch die Menge an freien Silberionen im Elektrolyten reduziert wird und einer Passivierung der Arbeitselektrode entgegen gewirkt werden kann, was ebenfalls zu einer verbesserten Lebensdauer des Sensors führt.

Die Größe der Partikel des partikelförmigen Materials kann entsprechend den jeweiligen Anforderungen variiert werden. Im Rahmen der vorliegenden Erfindung weisen die Partikel des partikelförmigen Materials üblicherweise einen mittleren Partikeldurchmesser von etwa 0,1 µm bis etwa 10 µm auf, wobei ein mittlerer Partikeldurchmesser von etwa 1,0 µm bis etwa 5,0 µm bevorzugt ist. In jedem Fall sollte die Partikelgröße des porösen Materials stets kleiner sein als die Schichtdicke der Elektrolytschicht, welche im Bereich von etwa 5 µm bis etwa 30 µm, bevorzugt im Bereich von etwa 10 µm bis etwa 20 µm, liegt.

Neben dem partikelförmigen Material umfasst die Elektrolytschicht als weitere Komponente mindestens ein Bindemittel. Bevorzugt handelt es sich bei dem Bindemittel um ein organisches Bindemittel, wie beispielsweise ein vernetztes oder unvernetztes, lineares oder verzweigtes organisches Polymer. Stärker bevorzugt umfasst das in der Elektrolytschicht enthaltene organische Bindemittel ein beliebiges vernetztes oder unvernetztes synthetisches Polymer, welches vorzugsweise aus der Gruppe bestehend aus einem Phenolharz, einem Epoxidharz, einem Vinylharz, einem PVC-Copolymer, einem Zweikomponenten-Epoxidharz, einem Polyurethanharzsystem und einem UV-härtbaren Polyacrylat-Momomergemisch ausgewählt ist. Besonders bevorzugt wird als vernetztes oder unvernetztes synthetisches Polymer ein Phenolharz, insbesondere ein vernetztes Phenolharz, eingesetzt.

Das bevorzugt nicht-quellbare Bindemittel ist mengenmäßig so bemessen, dass es die Partikel des anorganischen oder organischen partikelförmigen Materials zu einer porösen, nicht-quellbaren Gerüststruktur verbindet. Dies bedeutet, dass das Bindemittel die Zwischenräume zwischen den Partikeln des partikelförmigen Materials nicht vollständig ausfüllt, so dass sich eine nicht-quellbare Gerüststruktur bildet, welche Poren mit einem Durchmesser von etwa 500 nm bis etwa 5 µm, bevorzugt von etwa 1 µm bis etwa 3 µm, aufweist. Erfindungsgemäß bewirkt die poröse, nicht-quellbare Gerüststruktur eine relative Flüssigkeitsaufnahme von etwa 20 Gew.-% bis etwa 50 Gew.-%, bevorzugt von etwa 26 Gew.% bis etwa 44 Gew.-%, bezogen auf das Trockengewicht der porösen, nicht-quellbaren Gerüststruktur.

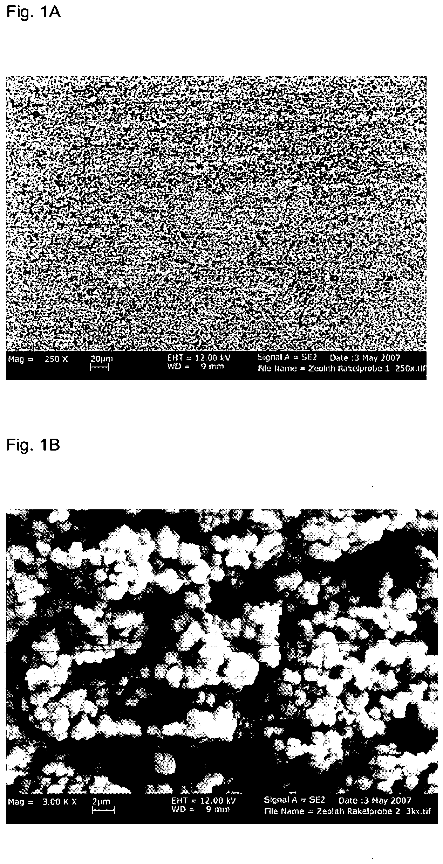

Die erforderlichen Mengen an Bindemittel können in Abhängigkeit von der Art und Menge des verwendeten partikelförmigen Materials sowie der gewünschten Porengröße variieren und von einem Fachmann den jeweiligen Anforderungen angepasst werden. Abb. 1a und 1b zeigen jeweils elektronenmikroskopische Aufnahmen der Elektrolytschicht eines erfindungsgemäßen Sensors, in welcher Partikel eines partikelförmigen Materials unter Verwendung eines Bindemittels zu einer porösen, nicht-quellbaren Gerüststruktur verbunden sind. Die poröse, nicht-quellbare Gerüststruktur weist eine regelmäßige Verteilung der Poren auf und besteht aus Einheiten, die in der Regel etwa 1 bis 3 Partikel breit und kettenartig verbunden sind.

Erfindungsgemäß kann die Elektrolytschicht neben dem mindestens einen partikelförmigen Material und dem mindestens einen Bindemittel ferner einen oder mehrere Hilfsstoffe umfassen. Hilfsstoffe, welche zu diesem Zweck verwendet werden können, umfassen insbesondere synthetische Cellulosederivate wie beispielsweise Alkylcellulosen, wobei der Begriff "Alkyl" für einen geradkettigen oder verzweigten Kohlenwasserstoffrest mit 1 bis 6 Kohlenstoffatomen steht. Bevorzugte Alkylcellulosen in Sinne der vorliegenden Erfindung sind aus der Gruppe bestehend aus Methylcellulose, Ethylcellulose, Propylcellulose, Ethylmethylcellulose und Propylmethylcellulose ausgewählt. Durch die Zugabe kleiner Mengen dieser Alkylcellulosen kann die Benetzungsfähigkeit der Strukturoberfläche mit dem flüssigen Elektrolyten verbessert werden. Weitere geeignete Hilfsstoffe umfassen beispielsweise Entschäumer.

Der flüssige Elektrolyt, welcher für eine leitende Elektrolytverbindung zwischen der Arbeitselektrode und der Gegenelektrode in dem erfindungsgemäßen elektrochemischen Sensor sorgt, kann ein beliebiger Elektrolyt, z.B. ein Elektrolyt auf Basis von Wasser, sein, welcher beim Anlegen einer Spannung durch gerichtete Bewegung von Ionen einen Transport von Ladungsträgern bewirkt. Im Rahmen der vorliegenden Erfindung ist es bevorzugt, dass der flüssige Elektrolyt ein Alkalimetallchlorid und ein pH-Puffersystem beinhaltet.

Als Alkalimetallchlorid, welches in einem elektrochemischen Sensor gemäß der vorliegenden Erfindung als Leitsalzkomponente des flüssigen Elektrolyten dient, können insbesondere Natrium- oder/und Kaliumchlorid verwendet werden. Die Verwendung von Natrium- oder/und Kaliumchlorid als Hauptkomponente des Elektrolyten bietet den Vorteil, dass die bei der Verwendung silberhaltiger Gegenelektroden aus der Gegenelektrode freigesetzten Silberionen infolge Silberchloridfällung zusätzlich an einer Wanderung in Richtung der Arbeitselektrode gehindert werden, wodurch die Ablagerung von elementarem Silber auf der Arbeitselektrode reduziert werden kann.

pH-Puffersysteme, welche für die flüssige Elektrolytlösung verwendet werden können, umfassen beliebige Puffersysteme, welche im Fachbereich bekannt sind und der Stabilisierung des pH-Werts in einem flüssigen Medium dienen. Beispielhafte pH-Puffersysteme umfassen, sind jedoch nicht beschränkt auf, Bicarbonatpuffer, Phosphatpuffer und dergleichen.

In einer bevorzugten Ausführungsform umfasst der flüssige Elektrolyt neben dem Alkalimetallchlorid und dem pH-Puffersystem weiterhin mindestens ein wasserlösliches Polymer. Als wasserlösliches Polymer kann ein beliebiges Polymer verwendet werden, welches durch Einbringen in die vorstehend beschriebene flüssige Elektrolytlösung eine Erhöhung der Viskosität der Flüssigkeit bewirkt und damit ebenfalls zur Reduzierung der Wanderungsgeschwindigkeit freigesetzter Ionen, beispielsweise freigesetzter Silberionen, beitragen kann. Wasserlösliche Polymere, welche im Rahmen der vorliegenden Erfindung Anwendung finden, sind beispielsweise Polyethylenglykol, Polyvinylpyrrolidon und Alkylpolyglykoside, wobei die Elektrolytflüssigkeit vorzugsweise Polyethylenglykol oder/und Polyvinylpyrrolidon als wasserlösliches Polymer beinhaltet. Besonders vorteilhaft kann eine derartige Elektrolytflüssigkeit in Sensoren zur Bestimmung von Sauerstoff eingesetzt werden.

In einer alternativen Ausführungsform kann die Elektrolytflüssigkeit neben dem Alkalimetallchlorid und dem pH-Puffersystem zusätzlich ein Enzym umfassen, welches eine gewünschte Umsetzung, wie beispielsweise die Hydratisierung eines vorbestimmten Substrates, beschleunigt. Ein im Rahmen der vorliegenden Erfindung besonders bevorzugtes Enzym ist Carboanhydrase, welches in Gegenwart von Wasser die Hydratisierung von Kohlendioxid zu Hydrogencarbonat katalysiert. Ein derartiger Elektrolyt bietet sich insbesondere für Elektroden vom Severinghaus-Typ an, bei denen die Bestimmung von Kohlendioxid auf einer pH-Messung des Innenelektrolyten beruht.

Im Rahmen der vorliegenden Erfindung ist es weiterhin bevorzugt, dass die Elektrolytschicht die nicht-flüchtigen Bestandteile des flüssigen Elektrolyten vor Inbetriebnahme des elektrochemischen Sensors umfasst. Hierzu kann eine diese Bestandteile enthaltende Flüssigkeit, vorzugsweise der flüssige Elektrolyt, z.B. durch Auftropfen, in die Poren der nicht-quellbaren Gerüststruktur eingebracht und nach Entfernen eines Überstandes an Elektrolytflüssigkeit, z.B. durch Abtupfen, für einen Zeitraum von mehreren Stunden, z.B. für einen Zeitraum von 48 Stunden oder mehr, bei einer Temperatur > 25 °C, z.B. bei einer Temperatur von 65 °C, gelagert werden, um ein Abdampfen der flüchtigen Bestandteile des flüssigen Elektrolyten zu ermöglichen. Auf diese Weise kann sichergestellt werden, dass nach dem Trockenschritt die nicht-flüchtigen Bestandteile des Elektrolyten, wie beispielsweise Alkalimetallchloride, Bestandteile der pH-Puffersysteme sowie weitere Substanzen, welche u.a. wasserlösliche Polymere oder Enyzme umfassen, in der Elektrolytschicht des elektrochemischen Sensors vorliegen.

Alternativ kann eine die nicht-flüchtigen Elektrolytbestandteile enthaltende Flüssigkeit bereits vor Ausbildung der pörosen, nicht-quellbaren Gerüststruktur einem das mindestens eine partikelförmige Material, das mindestens eine Bindemittel und gegebenenfalls weitere Substanzen enthaltenden Gemisch, welches beispielsweise in Form einer Paste vorliegt, zugesetzt werden. Das auf diese Weise erhaltene Gemisch kann im Anschluss ausgehärtet werden, wobei eine poröse, nicht-quellbare Gerüststruktur ausgebildet wird, deren Poren die nicht flüchtigen Bestandteile des flüssigen Elektrolyten enthalten. Diese Vorgehensweise bietet gegenüber dem oben beschriebenen Verfahren, d.h. gegenüber einer Einbringung des flüssigen Elektrolyten in die Poren einer bereits bestehenden Gerüststruktur, den Vorteil, dass die verschiedenen Bestandteile des flüssigen Elektrolyten einfacher, homogener und besser reproduzierbar in die poröse, nicht-quellbare Gerüststruktur des erfindungsgemäßen Sensors eingebracht werden können.

Die gaspermeable Deckschicht erlaubt das Eindringen des (unter Normalbedingungen) gasförmigen Analyten in den elektrochemischen Sensor, soll jedoch insbesondere den Eintritt von Ionen oder/und von nicht-flüchtigen Bestandteilen des wässrigen Messmediums verhindern. In einer bevorzugten Ausführungsform ist die gaspermeable Deckschicht daher für Ionen und nicht-flüchtige Bestandteile des wässrigen Messmediums undurchlässig.

Die gaspermeable Deckschicht kann aus einem beliebigen Material bestehen, welches für derartige Zwecke in Frage kommt. Bevorzugt umfasst die gaspermeable Deckschicht mindestens ein polymeres Material, wobei sich Siloxane, Polysulfone oder/und Polytetrafluorethylen als besonders vorteilhaft erwiesen haben. Als Siloxan, welches im Rahmen der vorliegenden Erfindung von Nutzen ist, kann beispielsweise ein oximvernetzender Silikonkautschuk verwendet werden, wie er unter der Handelsbezeichnung DELO-GUM® SI480 (Fa. DELO, Deutschland) kommerziell erhältlich ist. Geeignete Polysulfone umfassen insbesondere Polysulfone mit längeren Alkylketten, wie beispielsweise C₁₆-Alkylketten. Ein derartiges Produkt ist unter dem Handelsnamen BIOBLAND (Fa. ANATRACE, USA) bekannt.

Die Erzeugung der gaspermeablem Deckschicht kann auf unterschiedliche Weise erfolgen. Ein bevorzugtes Verfahren besteht in der Verwendung vorgefertigter Deckschichten, welche auf den elektrochemischen Sensor aufgebracht werden. Eine Fixierung der Membran auf dem Sensor kann hierbei mittels beliebiger Techniken erfolgen, wobei Verklebung oder Laserverschweißung als bevorzugt anzusehen sind.

Alternativ besteht die Möglichkeit, die gaspermeable Deckmembran *in situ* zu erzeugen, indem eine Lösung eines geeigneten gaspermeablen Polymers oder Prepolymers auf den elektrochemischen Sensor aufgebracht und anschließend getrocknet wird. Das Aufbringen des Polymers auf den elektrochemischen Sensor erfolgt bevorzugt durch Aufsprühen, Tauchcoaten, Dispergieren oder Siebdrucken einer vorzugsweise verdünnten Lösung des Polymers oder Prepolymers, ist jedoch nicht auf diese Verfahren beschränkt. Als Lösungsmittel wird bevorzugt ein organisches Lösungsmittel, insbesondere ein organisches Lösungsmittel mit einem Siedepunkt von ≤ 100 °C, eingesetzt, wobei die Wahl eines geeigneten Lösungsmittels grundsätzlich je nach den Eigenschaften des verwendeten Polymers oder Prepolymers und/oder der Auftragetechnik durch einen Fachmann erfolgen kann.

Die auf diese Weise erhaltenen und in einem elektrochemischen Sensor gemäß der vorliegenden Erfindung eingesetzten gaspermeablen Deckschichten weisen üblicherweise eine Dicke von etwa 5,0 µm bis etwa 30 µm, bevorzugt von etwa 8,0 µm bis etwa 15 µm, auf.

Die Arbeitselektrode und Gegenelektrode des erfindungsgemäßen elektrochemischen Sensors können aus einem beliebigen Material bestehen, welches für die Zwecke der vorliegenden Erfindung geeignet ist. Die Arbeitselektrode ist vorzugsweise aus einem Verbundmaterial auf Basis von Gold oder Rutheniumoxid ausgebildet, wobei sich ein Verbundmaterial auf Basis von Gold insbesondere für Sensoren zur amperometrischen Bestimmung von Sauerstoff und ein Verbundmaterial auf Basis von Rutheniumoxid insbesondere für Sensoren zur potentiometrischen Bestimmung von Kohlendioxid mittels einer pH-Elektrode nach dem Severinghaus-Prinzip eignet. Demgegenüber ist die Gegenelektrode eines erfindungsgemäßen Sensors bevorzugt aus einem Verbundmaterial auf Basis von Silber oder Silber/Silberchlorid ausgebildet. Hierbei hat sich ein Verbundmaterial auf Basis von Silber insbesondere für Gegenelektroden in Sensoren zur Bestimmung von Sauerstoff als vorteilhaft erwiesen, während ein Verbundmaterial auf Basis von Silber/Silberchlorid bevorzugt für Gegenelektroden in Sensoren zur Bestimmung von Kohlendioxid angewendet werden kann. Die Begriffe "Arbeitselektrode" und "Messelektrode" bzw. die Begriffe "Gegenelektrode" und "Referenzelektrode", wie sie im Fachbereich jeweils überwiegend für entweder amperometrische Elektroden oder potentiometrische Elektroden geläufig sind, werden im Rahmen dieser Anmeldung synonym verwendet.

Das leitfähige Elektrodenmaterial, welches zur Herstellung der Elektrodenmatrix beispielsweise in Form einer Paste bereitgestellt werden kann, umfasst neben dem Metall oder Metalloxid sowohl im Falle der Arbeitselektrode bzw. Messelektrode als auch im Falle der Gegenelektrode bzw. Referenzelektrode vorzugsweise ein nicht-leitendes polymeres Bindemittel, wobei Vinylharze besonders bevorzugt sind.

Bevorzugt ist der erfindungsgemäße elektrochemische Sensor für eine Mehrfachmessung des zu bestimmenden Analyten ausgebildet. Dies ist insbesondere in Anwendungen erwünscht, in welchen mit einem Sensor eine Vielzahl von Proben gemessen werden soll oder in welchen eine stetige, d.h. kontinuierliche oder diskontinuierliche Kontrolle des Vorhandenseins oder/und der Menge eines Analyten über einen längeren Zeitraum von z.B. 1 Tag oder länger, insbesondere 1 Woche oder länger, vorgenommen werden soll. In einer bevorzugten Ausführungsform sieht die Erfindung demnach vor, dass der elektrochemische Sensor als Messzelle ausgebildet ist, durch die eine den gasförmigen Analyten enthaltende Probenflüssigkeit hindurchgeleitet wird und welche beispielsweise Teil eines Analysators ist.

Der erfindungsgemäße elektrochemische Sensor kann zur Bestimmung eines in einem wässrigen Messmedium, welches aus einer beliebigen Quelle stammen kann, gelösten gasförmigen Analyten eingesetzt werden. In einer bevorzugten Ausführungsform dient der elektrochemische Sensor zur Bestimmung eines in einer Körperflüssigkeit gelösten gasförmigen Analyten, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, wobei Vollblut, Plasma und Serum als besonders bevorzugt anzusehen sind. Der qualtitativ oder/und quantitativ zu bestimmende gasförmige Analyt ist bevorzugt aus Sauerstoff und Kohlendioxid ausgewählt.

In einem weiteren Aspekt betrifft die vorliegende Erfindung ein Verfahren zur Herstellung eines erfindungsgemäßen elektrochemischen Sensors, umfassend die Schritte:
(a) Bereitstellen mindestens eines partikelförmigen Materials,
(b) Vermischen des partikelförmigen Materials mit mindestens einem Bindemittel und gegebenenfalls weiteren Substanzen,
(c) Verarbeiten des in Schritt (b) erhaltenen Gemisches zu einer Paste,
(d) Aufbringen der in Schritt (c) erhaltenen Paste auf einen Träger,
(e) Aushärten der auf den Träger aufgebrachten Paste, wobei eine poröse, nicht-quellbare Gerüststruktur ausbildet wird, deren Poren einen Durchmesser von 500 nm bis etwa 5 µm, insbesondere von 1 µm bis etwa 3 µm aufweisen oder/ und die poröse nicht-quellbare Gerüststruktur eine relative Flüssigkeitsaufnahme von etwa 20 Gew.-% bis etwa 50 Gew.-% bevorzugt von etwa 26 Gew.-% bis etwa 44 Gew.-%, bezogen auf das Trockengewicht der porösen, nicht-quellbaren Gerüststruktur, bewirkt und
(f) Anfertigen eines elektrochemischen Sensors, der die in Schritt (e) erhaltene poröse, nicht-quellbare Gerüststruktur als Elektrolytschicht, sowie eine Arbeitselektrode, eine Gegenelektrode und eine gaspermeable Deckschicht enthält.

Zum Zwecke der Herstellung der erfindungsgemäßen elektrochemischen Sensoren wird mindestens ein anorganisches oder organisches partikelförmiges Material mit mindestens einem anorganischen oder organischen Bindemittel, welche jeweils wie vorstehend definiert sind, und gegebenenfalls weiteren Substanzen vermischt und zu einer Paste verarbeitet. Nach Aufbringen der Paste auf einen Träger und anschließendes Aushärten der Paste wird eine poröse, nicht-quellbare Gerüststruktur ausgebildet, welche als Elektrolytschicht in einem elektrochemischen Sensor gemäß der vorliegenden Erfindung verwendet werden kann. Dieser enthält neben der porösen Elektrolytschicht eine Arbeitselektrode, eine Gegenelektrode und eine gaspermeable Deckschicht. Zur Durchführung des vorstehend beschriebenen Verfahrens kann die Paste beispielsweise mittels Siebdruck oder Rakeltechniken auf einen Träger aufgebracht werden. So kann der Träger in einer Ausführungsform der Erfindung beispielsweise sowohl die Arbeitselektrode als auch die Gegenelektrode bereits vor Aufbringen der Paste umfassen. Alternativ ist es allerdings ebenso möglich, die Arbeitselektrode und die Gegenelektrode erst nach Aufbringen und Aushärten der Paste auf den Träger aufzubringen. Das erfindungsgemäße Herstellungsverfahren erweist sich insofern als vorteilhaft, da sowohl die Paste als auch die Elektroden mittels Siebdruck und damit in dünner und definierter Schichtstärke auf den Träger aufgebracht werden können.

Bevorzugt umfasst das erfindungsgemäße Verfahren ferner das Einbringen einer nicht-flüchtige Elektrolytbestandteile enthaltenden Flüssigkeit, z.B. eines flüssigen Elektrolyten, in die poröse, nicht-quellbare Gerüststruktur und anschließendes Abdampfen deren flüchtiger Bestandteile. Aus diesen nicht-flüchtigen Bestandteilen kann der flüssige Elektrolyt gebildet werden, welcher für die elektrisch leitende Verbindung zwischen der Arbeitselektrode und der Gegenelektrode in dem erfindungsgemäßen Sensor sorgt. Die Flüssigkeit kann hierbei unter Verwendung von Techniken, wie sie im Zusammmenhang mit der Erläuterung des elektrochemischen Sensors beschrieben sind, in die Poren der nicht-quellbaren Gerüststruktur eingebracht werden.

Alternativ kann eine die nicht-flüchtigen Elektrolytbestandteile enthaltende Flüssigkeit bereits vor Ausbildung der pörosen, nicht-quellbaren Gerüststruktur in ein Gemisch eingebracht werden,das mindestens ein partikelförmiges Material mit mindestens einem Bindemittel und gegebenenfalls weitere Substanzen enthält, wodurch auf einfache Weise eine gleichmäßige Verteilung der festen Bestandteile des Elektrolyten in der nach Aushärten des Gemisches erhaltenen porösen, nicht-quellbaren Gerüststruktur erzielt werden kann. In einer bevorzugten Ausführungsform der Erfindung umfasst das erfindungsgemäße Verfahren das Einbringen der Flüssigkeit in eine Paste, wobei die Flüssigkeit der Paste insbesondere vor Aufbringen derselben auf einen geeigneten Träger zugesetzt werden kann.

In dieser Ausführungsform wird eine Flüssigkeit zugegeben, welche die nicht-flüchtigen Elektrolytbestandteile vorzugsweise in einem mit dem Bindemittel mischbaren Lösungsmittel enthält. Dieses Lösungsmittel kann beispielsweise ein Glykol wie etwa Ethylenglykol, Propylenglykol oder ein Gemisch davon, das gegebenenfalls bis zu 20 % (v/v) Wasser enthalten kann, sein.

In einer bevorzugten Ausführungsform umfasst die Inbetriebnahme des erfindungsgemäßen elektrochemischen Sensors ferner dessen vorherige Aktivierung unter Einsatz geeigneter Mittel. Die Aktivierung kann beispielsweise durch Inkontaktbringen des Sensors mit einer geeigneten Flüssigkeit, insbesondere mit einer wässrigen Flüssigkeit, bewirkt werden. Hierbei tritt die zur Aktivierung des Sensors dienende Flüssigkeit, insbesondere Wasser, beispielsweise durch isotherme Destillation durch die gaspermeable Deckschicht des elektrochemischen Sensors hindurch und kondensiert in den Poren der nicht-quellbaren Gerüststruktur, wobei die vorab eingebrachten festen Bestandteile der Elektrolytflüssigkeit gelöst werden.

Zur Aktivierung des Sensors bevorzugte Flüssigkeiten weisen einen osmotischen Druck auf, welcher dem osmotischen Druck der in der porösen, nicht-quellbaren Gerüststruktur des Sensors aufgenommenen Elektrolytflüssigkeit entspricht oder diesem ähnlich ist, und umfassen insbesondere wässrige Kalibrier- und Kontrollflüssigkeiten, wie z. B. Puffersysteme oder Salzlösungen mit einer Salzkonzentration von etwa 100 bis etwa 200 mmol/l oder Osmolaritätswerten von etwa 200 mosmol/l bis etwa 500 mosmol/l. Ist der zu bestimmende Analyt in einer wässrigen Flüssigkeit enthalten, so kann der Sensor gegebenenfalls auch mittels des wässrigen Messmediums aktiviert werden.

Unter Verwendung dieses Verfahrens können eine dauerhafte Lagerfähigkeit und eine hohe "in-use"-Lebensdauer des elektrochemischen Sensors sichergestellt werden. Alternativ sieht die vorliegende Erfindung allerdings auch vor, dass die Elektrolytschicht den Elektrolyten vor Inbetriebnahme des elektrochemischen Sensors bereits in flüssiger Form enthält.

In noch einem weiteren Aspekt betrifft die vorliegende Erfindung ein Verfahren zur Bestimmung eines in einem wässrigen Messmedium gelösten gasförmigen Analyten, umfassend die Schritte:
(a) Inkontaktbringen des wässrigen Messmediums mit einem erfindungsgemäßen elektrochemischen Sensor, und
(b) Bestimmen des in dem wässrigen Messmedium gelösten gasförmigen Analyten durch Messung eines von dem elektrochemischen Sensor erzeugten Signals.

Zur Bestimmung des gasförmigen Analyten kann der erfindungsgemäße elektrochemische Sensor in einer beliebigen Art und Weise ausgestaltet sein, welche einen Kontakt zwischen dem elektrochemischen Sensor und dem wässrigen Messmedium ermöglicht. So kann der Sensor beispielsweise als Messzelle ausgebildet sein, durch welche das den gasförmigen Analyten enthaltende wässrige Messmedium hindurchgeleitet wird. Ferner kann der erfindungsgemäße Sensor zusammen mit weiteren Sensoren, welche beispielsweise der Bestimmung unterschiedlicher "Point-of-Care"-Parameter dienen, in austauschbaren Messkammern (Sensor-Kassetten) ausgebildet sein.

Abhängig vom Vorhandensein oder/und der Menge des Analyten wird vom Sensor ein messbares Signal erzeugt. Bevorzugt handelt es sich bei diesem Signal um ein elektrisches Signal, wie beispielsweise elektrischen Strom, Spannung, Widerstand, etc., welches unter Verwendung geeigneter Mittel ausgewertet bzw. ausgelesen wird. Vorzugsweise ist der elektrochemische Sensor ein amperometrischer Sensor, wie beispielsweise im Falle eines Sauerstoffsensors, oder ein potentiometrischer Sensor, wie beispielsweise im Falle eines Kohlendioxidsensors.

Die Erfindung soll durch die nachfolgenden Figuren und Beispiele näher erläutert werden.

### Abbildungen

Fig.1a und Fig. 1b zeigen elektronenmikroskopische Aufnahmen der porösen Elektrolytschicht in einem elektrochemischen Sensor gemäß der vorliegenden Erfindung.

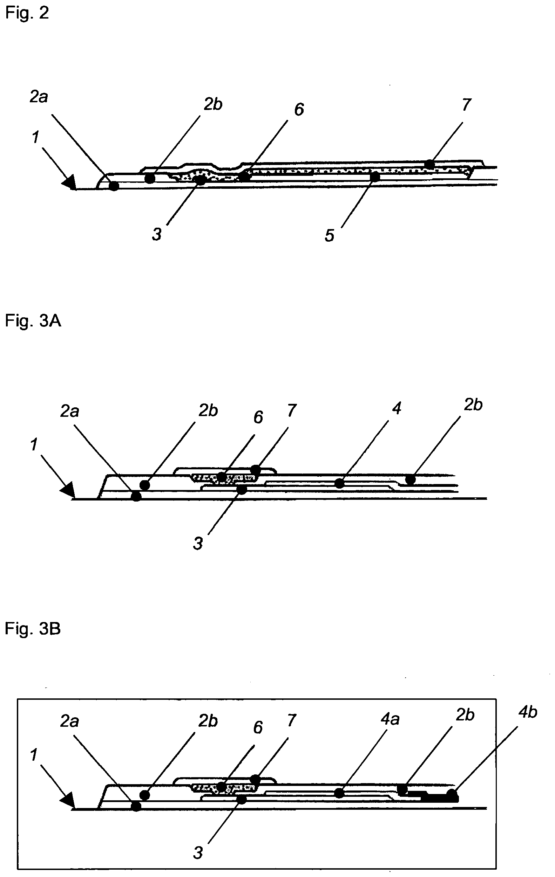

Fig. 2 zeigt einen Schnitt durch den Kanalbereich in einem elektrochemischen pO₂-Sensor gemäß der vorliegenden Erfindung. Als Träger (1) dient ein mit einer dünnen Schicht aus Polycarbonat beschichtetes Plättchen aus einer Al/Mg-Legierung, auf welches eine Gassperrschicht (2a) aufgebracht ist, welche ihrerseits der Minimierung bzw. vollständigen Unterbindung des Eindiffundierens von Gasen in die Polycarbonatschicht bzw. des Abdiffundierens von Gasen aus der Polycarbonatschicht dient. Auf die Gassperrschicht (2a) ist eine Isolierschicht (2b) aufgebracht, welche dazu vorgesehen ist, Störpotentiale/Ströme bei Flüssigkontakt zu vermeiden. Im Messbereich ist ein Fenster ausgespart, welches mit einer gaspermeablen Deckschicht (7) verschlossen ist. Der elektrochemische pO₂-Sensor an sich besteht aus der Arbeitselektrode (3), der Gegenelektrode (5), der porösen Elektrolytschicht (6), welche längs des Messkanals verläuft und die aktiven Flächen sowohl der Arbeitselektrode (3) als auch der Gegenelektrode (5) vollständig überdeckt, sowie der gaspermeablen Deckschicht (7), welche ihrerseits die poröse Elektrolytschicht (6) vollständig überdeckt.
Fig. 3a zeigt einen Längsschnitt durch die Arbeitselektrode in einem elektrochemischen pO₂-Sensor gemäß der vorliegenden Erfindung. Die Arbeitselektrode (3) besteht vorzugsweise aus einem Verbundmaterial auf Basis von Gold, welches weiterhin eine polymere Komponente enthält. Die Arbeitselektrodenableitung (4), welche mit einem Ende die Arbeitselektrode (3) flächig kontaktiert und mit einem zweiten Ende einen offenen Kontakt für eine elektrische Steckverbindung bildet, kann beispielsweise aus einem dünnen, länglich ausgeführten Verbundmaterial auf Basis von Silber bestehen. Die übrigen Bezugszeichen besitzen jeweils die in Fig. 2 angegebene Bedeutung.
Fig. 3b zeigt einen Längsschnitt durch die Arbeitselektrode mit alternativer Silber-Brückenschicht und Silber/Silberchlorid-Ableitung in einem elektrochemischen pO₂-Sensor gemäß der vorliegenden Erfindung. Hierbei liegt in Abänderung zu Fig. 3a lediglich noch eine Brückenschicht (2a) vor, welche außerhalb des Messbereiches auf eine Silber/Silberchlorid-Ableitung (4b) übergeht. Die Ableitung (4b) kann beispielsweise mittels Siebdruck aus einer entsprechenden Siebdruckpaste hergestellt werden. Die übrigen Bezugszeichen besitzen jeweils die in Fig. 2 angegebene Bedeutung.

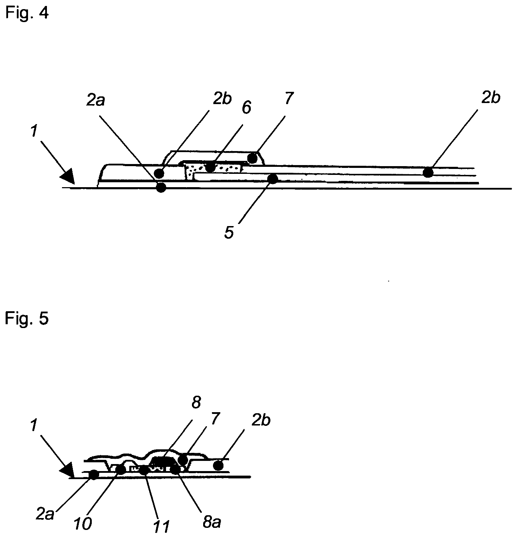

Fig.4 zeigt einen Längsschnitt durch die Gegenelektrode in einem elektrochemischen O₂-Sensor gemäß der vorliegenden Erfindung. Die Gegenelektrode (5), welche beispielsweise mittels Siebdruck hergestellt werden kann, besteht vorzugsweise aus einem Verbundmaterial auf Basis von Silber, welches weiterhin eine polymere Komponente enthält. Die übrigen Bezugszeichen besitzen jeweils die in Fig. 2 angegebene Bedeutung.
Fig. 5 zeigt einen Schnitt durch den Kanalbereich in einem elektrochemischen CO₂-Sensor gemäß der vorliegenden Erfindung. Der elektrochemische CO₂-Sensor an sich besteht aus der Arbeitselektrode (8) (pH-sensitive Messelektrode), einer Brückenschicht (8a), der Gegenelektrode (Referenzelektrode) (10), einer porösen Elektrolytschicht (11) sowie einer gaspermeablen Deckschicht (7). Die Arbeits- bzw. pH-Elektrode (8), welche beispielsweise mittels Siebdruck generiert werden kann, besteht vorzugsweise aus einem Verbundmaterial auf Basis von Rutheniumoxid, welches weiterhin eine polymere Komponente enthält. Elektrisch abgeleitet wird diese Schicht aus dem unmittelbaren Messbereich durch eine beispielsweise aus einer Graphitpaste erzeugte Brückenschicht (8a). Die schichtartig aufgebaute Gegenelektrode (10) wird insbesondere mittels Siebdruck generiert und besteht vorzugsweise aus einem Verbundmaterial auf Basis von Silber/Silberchlorid. Die übrigen Bezugszeichen besitzen jeweils die in Fig. 2 angegebene Bedeutung.

### Beispiele

### Beispiel 1: Herstellung einer siebdruckfähigen Paste

In ein verschließbares Glas wurden 12,0 g Ethylcellulose-Pulver N 50 und 192,0 g Terpineol eingewogen und das Gemisch bis zum vollständigen Lösen der Ethylcellulose 2 Wochen bei Raumtemperatur gerührt. Zur Herstellung einer Menge von 100 g an gebrauchsfertiger, siebdruckfähiger Paste wurden im Anschluss 25,6 g der erhaltenen Ethylcellulose-Lösung, 32,3 g Phenolharz PR 373, 1,0 g des Entschäumers Byk 051 (Fa. Byk Chemie), 5,0 g Benzylalkohol und 2,9 g Butyldiglykol in ein Becherglas eingewogen und dieses Gemisch intensiv gerührt. Zu diesem Gemisch wurden 43,2 g des Zeoliths LZ-Y 52 (Fa. Sigma-Aldrich, Produkt-Nr. 334448) hinzugefügt und der Ansatz bis zur homogenen Verteilung des Füllstoffes intensiv gerührt, wobei eine siebdruckfähige Paste erhalten wurde.

### Beispiel 2: Bestimmung der relativen Flüssigkeitsaufnahme einer nach Beispiel 1 hergestellten und ausgehärteten Paste

Zur Bestimmung der relativen Flüssigkeitsaufnahme einer nach Beispiel 1 hergestellten Paste wurden mehrere Objektträger (76 x 26 mm, ISO Norm 8038/l, Fa. Roth) auf einer Analysenwaage ausgewogen (m_{Träger}). Durch Aufbringen der nach Beispiel 1 hergestellten Paste auf die Objektträger unter Verwendung einer 80 µm Spiralabziehrakel wurden anschließend Prüfschichten auf die Objektträgern aufgetragen. Die aufgetragene Paste bedeckte eine rechteckige Fläche von ungefähr 60 x 18 mm. Da diese Fläche variabel ist, wurde sie erst nach dem Aushärten der Paste exakt bestimmt. 10-15 Minuten nach dem Auftragen der Paste wurden die Prüfkörper im Trockenschrank für 4 h bei 110 °C ausgehärtet. Anschließend wurden die Prüfkörper in einem Exsikkator (über CaO) bis zur Messung aufbewahrt. Die Objektträger mit der ausgehärteten Paste wurden erneut gewogen (m_{trocken}) und unmittelbar danach in jeweils etwa 40 ml Flüssigkeit eingetaucht, die sich in verschraubbaren Plastikdosen mit einem Volumen von 100 ml befand. Während des Messablaufs blieben die Dosen mit den eingetauchten Prüfkörpern verschlossen. Zu definierten Zeitpunkten wurden die Prüfkörper aus den Dosen entnommen, abgetupft und gewogen, um die Menge an aufgenommener Flüssigkeit zu bestimmen. Die Messungen wurden abgebrochen, wenn die Menge an aufgenommener Flüssigkeit und damit die Masse (m_{feucht}) konstant blieb. Die Messungen wurden bei Raumtemperatur und Normaldruck durchgeführt. Aus den erhobenen Messwerten wurde für jeden Prüfkörper die relative Flüssigkeitsaufnahme als Mittelwert der entsprechenden Messwerte von mindestens 3 Prüfkörpern berechnet, mit einer Standardabweichung von maximal 1 %.

Die jeweilige relative Flüssigkeitsaufnahme wurde auf das jeweils zuvor bestimmte Trockengewicht der ausgehärteten Paste (jeweils abzüglich des Gewichts des Objektträgers) bezogen: (relative Flüssigkeitsaufnahme [%] = [(m_{feucht} - m_{Träger})/(mₜᵣₒₖₑₙ - m_{Träger}) - 1] * 100). Die Werte der relativen Flüssigkeitsaufnahme ändern sich entsprechend der Porosität der ausgehärteten Paste. Ausgehärtete Pasten mit einer unerwünscht hohen Porosität besitzen relative Flüssigkeitsaufnahmen zwischen 50 und 70 %, ausgehärtete Pasten mit einer unerwünscht niedrigen Porosität relative Flüssigkeitsaufnahmen von weniger als 20 %. Ausgehärtete Pasten, welche im Rahmen der vorliegenden Erfindung Anwendung finden, weisen eine Porosität auf, welche eine relative Flüssigkeitsaufnahme zwischen 20 Gew.-% und 50 Gew.-%, bevorzugt zwischen 26 Gew.-% und 44 Gew.-%, bewirkt.

### Beispiel 3: Herstellung eines Sensorhalbzeugs mit poröser Elektrolytschicht

Zur Herstellung eines Sensorhalbzeugs, welches als Bestandteil eines elektrochemischen Sensors gemäß der vorliegenden Erfindung verwendet werden kann, wurde die gemäß Beispiel 1 hergestellte Paste auf ein Sieb aufgetragen, mittels Siebdruck auf einen geeigneten Träger, umfassend eine Arbeitselektrode und eine Gegenelektrode, aufgebracht und bei erhöhter Temperatur zum Aushärten gebracht. Die Dicke der ausgehärteten Schicht betrug 10-20 µm.

### Beispiel 4: Einbringung eines für O₂-Sensoren geeigneten Elektrolyten in die Poren des nach Beispiel 3 hergestellten Sensorhalbzeugs

Um 100 g Innenelektrolyt für einen elektrochemischen O₂-Sensor gemäß der vorliegenden Erfindung herzustellen, wurden 0,098g Na₂HPO₄ (p.a.), 0,094 g KH₂PO₄ (p.a.), 0,257 g NaCl (p.a.), 0,060 g Polyethylenglykol 6000 (zur Synthese) und 0,744 g Glycerin (p.a.) in 98,75 g entionisiertem Wasser unter Erhalt einer pH-gepufferten Elektrolytlösung gelöst. Die Einbringung der Salze in die poröse Elektrolytschicht erfolgte mittels Exzessmethode. Hierzu wurde das gemäß Beispiel 3 hergestellte Sensorhalbzeug vor Einbringen der Elektrolytlösung zunächst für 1 h in einem Gefäß über entionisiertem Wasser bei 100 % relativer Luftfeuchtigkeit aufbewahrt. Anschließend wurde die Elektrolytlösung im Überschuss auf die nach oben offene, poröse Schicht des Sensors dispensiert und der Überschuss mit einem Filterpapier entfernt. In einem geschlossenen Gefäß wurde das auf diese Weise behandelte Sensorhalbzeug anschließend so lange bei 100% relativer Luftfeuchtigkeit über Wasser gelagert, bis sich die Poren der porösen Schicht mit der Elektrolytlösung gefüllt hatten (ca. 2 h). Nach Entfernen des Überstandes an Elektrolytlösung mit einem Filterpapier wurde das Sensorhalbzeug zum Zwecke des Abdunstens der flüchtigen Bestandteile für 72 h bei 65 °C gelagert.

### Beispiel 5: Einbringung eines für CO₂-Sensoren geeigneten Elektrolyten in die Poren des nach Beispiel 3 hergestellten Sensorhalbzeugs

Um 100 g Innenelektrolytlösung für einen elektrochemischen CO₂-Sensor gemäß der vorliegenden Erfindung herzustellen, wurden 0,252 g NaHCO₃ (p.a.) und 0,559 g KCl (p.a.) zusammen mit 0,15 g des Enzyms Carboanhydrase (Fa. Serva, Produkt-Nr. 15880) in 99,04 g entionisiertem Wasser gelöst. Die Einbringung der Elektrolytlösung in die poröse, nach Beispiel 3 hergestellte Schicht erfolgte analog Beispiel 4 mittels Exzessmethode.

### Beispiel 6: Herstellung eines für O₂-Sensoren geeigneten Sensorhalbzeugs mit poröser Elektrolytschicht

Eine alternative Ausführungsform zu den Beispielen 3 und 4 umfasst das Einbringen der nicht-flüchtigen Elektrolytbestandteile in einem geeigneten Lösungsmittel bereits in die siebdruckfähige Paste.

Zur Herstellung eines Sensorhalbzeugs, welches als Bestandteil eines elektrochemischen O₂-Sensors gemäß der vorliegenden Erfindung verwendet werden kann, wurden 0,120 g Na₂HPO₄ (p.a.), 0,110 g KH₂PO₄ (p.a.), 0,310 g NaCl (p.a.) und 0,066 g Polyethylenglykol 6000 (zur Synthese) in 1,6 g entionisiertem Wasser gelöst. Die auf diese Weise erhaltene Lösung wurde anschließend mit einer Lösung von 0,900 g Glycerin (p.a.) in 23,15 g Ethylengykol versetzt und bis zum Erhalt einer klaren Lösung gerührt.

Zur Herstellung des Sensorhalbzeugs wurden 4,2 g der wässrigen Ethylenglykollösung in 100 g der nach Beispiel 1 hergestellten Paste eingerührt, woraufhin die den flüssigen Elektrolyten enthaltende Paste mittels Siebdruck auf einen geeigneten Träger aufgebracht und bei erhöhter Temperatur zum Aushärten gebracht wurde. Ein anschließender Dispensierschritt ist nicht erforderlich.

### Beispiel 7: Herstellung eines für CO₂-Sensoren geeigneten Sensorhalbzeugs mit poröser Elektrolytschicht

Eine alternative Ausführungsform zu den Beispielen 3 und 5 umfasst das Einbringen der nicht-flüchtigen Elektrolytbestandteile in einem geeigneten Lösungsmittel bereits in die siebdruckfähige Paste.

Zur Herstellung eines Sensorhalbzeugs, welches als Bestandteil eines elektrochemischen CO₂-Sensors gemäß der vorliegenden Erfindung verwendet werden kann, wurden 0,150 g Na₂CO₃ (p.a.) und 0,250 g KCl (p.a.) in 1,6 g entionisiertem Wasser gelöst. Diese Lösung wurde alsdann mit 8 g Ethylengykol versetzt und bis zum Erhalt einer klaren Lösung gerührt.

Zur Herstellung des Sensorhalbzeugs wurden 2,69 g der wässrigen Ethylenglykollösung in 100 g der nach Beispiel 1 hergestellten Paste eingerührt, woraufhin die den flüssigen Elektrolyten enthaltende Paste mittels Siebdruck auf einen geeigneten Träger aufgebracht und bei erhöhter Temperatur zum Aushärten gebracht wurde. Ein anschließender Dispensierschritt ist nicht erforderlich.

### Beispiel 8: Aufbringung einer gaspermeablen Deckschicht auf das nach einem der Beispiele 4-7 hergestellte Sensorhalbzeug und Verpackung der gebrauchsfertigen Sensorplättchen

Die Aufbringung einer Deckschicht auf das gemäß einem der Beispiele 4-7 hergestellte Sensorhalbzeug erfolgte mittels Siebdruck. Hierzu wurde ein oximspaltendes Silikon mittels Siebdruck auf das Sensorhalbzeug aufgetragen und bei erhöhter Temperatur zum Aushärten gebracht. Die Dicke der ausgehärteten Schicht betrug ca. 10 µm. Die auf diese Weise erhaltenen Sensorplättchen wurden anschließend zum Zwecke der Aufbewahrung bis zum Gebrauch in einem geeigneten Gebinde luftdicht verpackt.

## Patentansprüche

1. Elektrochemischer Sensor zur Bestimmung eines in einem wässrigen Messmedium gelösten gasförmigen Analyten, umfassend:
(a) eine Arbeitselektrode (3) und eine Gegenelektrode (4),
(b) eine zwischen der Arbeitselektrode (3) und der Gegenelektrode (4) lokalisierte Elektrolytschicht (6),
(c) eine gaspermeable Deckschicht (7), welche zur räumlichen Abtrennung der Arbeitselektrode (3), der Gegenelektrode (4) und der Elektrolytschicht (6) vom wässrigen Messmedium dient,
**dadurch gekennzeichnet,**
**dass** die Elektrolytschicht (6) mindestens ein partikelförmiges Material und mindestens ein Bindemittel umfasst, welche zusammen eine poröse, nicht-quellbare Gerüststruktur ausbilden, wobei die Poren der nicht-quellbaren Gerüststruktur zur Aufnahme eines flüssigen Elektrolyten vorgesehen sind oder diesen enthalten, und
(i) die Poren der nicht-quellbaren Gerüststruktur einen Durchmesser von etwa 500 nm bis etwa 5 µm, insbesondere von etwa 1 µm bis etwa 3 µm aufweisen, oder/und
(ii) die poröse, nicht-quellbare Gerüststruktur eine relative Flüssigkeitsaufnahme von etwa 20 Gew.-% bis etwa 50 Gew.-%, bevorzugt von etwa 26 Gew.-% bis etwa 44 Gew.-%, bezogen auf das Trockengewicht der porösen, nicht-quellbaren Gerüststruktur, bewirkt.

2. Elektrochemischer Sensor nach Anspruch 1,
**dadurch gekennzeichnet,**
**dass** das partikelförmige Material (i) ein anorganisches partikelförmiges Material ausgewählt aus der Gruppe bestehend aus Controlled Porous Glass (CPG), Kieselgelen und Silikaten ist, oder/und (ii) ein natürlich vorkommendes oder synthetisches Alumosilikat, insbesondere ein synthetisches Alumosilikat, ist, oder/und (iii) ein Zeolith, insbesondere Faujasit, ist.

3. Elektrochemischer Sensor nach Anspruch 1 oder 2,
**dadurch gekennzeichnet,**
**dass** das partikelförmige Material Kanäle, insbesondere Kanäle mit einem Durchmesser zwischen etwa 0.1 nm bis etwa 10 nm, und optional lonentauschergruppen aufweist.

4. Elektrochemischer Sensor nach einem der Ansprüche 1 bis 3,
**dadurch gekennzeichnet,**
**dass** die Partikel des partikelförmigen Materials einen mittleren Partikeldurchmesser von etwa 0,1 µm bis etwa 10 µm, insbesondere von etwa 1,0 µm bis etwa 5,0 µm, aufweisen.

5. Elektrochemischer Sensor nach einem der Ansprüche 1 bis 4,
**dadurch gekennzeichnet,**
**dass** das Bindemittel ein organisches Bindemittel ist, wobei das organische Bindemittel gegebenenfalls ein vernetztes oder unvernetztes synthetisches Polymer umfasst und das vernetzte oder unvernetzte synthetische Polymer gegebenenfalls eine Verbindung ausgewählt aus der Gruppe bestehend aus einem Phenolharz, einem Epoxidharz, einem Vinylharz, einem PVC-Copolymer, einem Zweikomponenten-Epoxidharz, einem Polyurethanharzsystem und einem UV-härtbaren Polyacrylat-Momomergemisch, insbesondere ein Phenolharz, ist.

6. Elektrochemischer Sensor nach einem der Ansprüche 1 bis 5,
**dadurch gekennzeichnet,**
**dass** die Elektrolytschicht (6) eine Dicke von etwa 5 µm bis etwa 30 µm, insbesondere von etwa 10 µm bis etwa 20 µm, aufweist.

7. Elektrochemischer Sensor nach einem der Ansprüche 1 bis 6,
**dadurch gekennzeichnet,**
**dass** der flüssige Elektrolyt (i) ein Alkalimetallchlorid und ein pH-Puffersystem, und (ii) gegebenenfalls ein wasserlösliches Polymer, insbesondere Polyethylenglykol oder/und Polyvinylpyrrolidon, oder ein Enzym, insbesondere Carboanhydrase, umfasst.

8. Elektrochemischer Sensor nach einem der Ansprüche 1 bis 7,
**dadurch gekennzeichnet,**
**dass** die Elektrolytschicht (6) die nicht-flüchtigen Bestandteile des flüssigen Elektrolyten vor Inbetriebnahme des Sensors umfasst.

9. Elektrochemischer Sensor nach einem der Ansprüche 1 bis 8,
**dadurch gekennzeichnet,**
**dass** die gaspermeable Deckschicht (i) für Ionen und nicht-flüchtige Bestandteile des wässrigen Messmediums undurchlässig ist, oder/und (ii) ein Siloxan, ein Polysulfon oder/und Polytetrafluorethylen umfasst, oder/und (iii) eine Dicke von etwa 5,0 µm bis etwa 30 µm, bevorzugt von etwa 8,0 µm bis etwa 15 µm, aufweist.

10. Verfahren zur Herstellung eines elektrochemischen Sensors nach einem der Ansprüche 1 bis 9, umfassend die Schritte:
(a) Bereitstellen mindestens eines partikelförmigen Materials,
(b) Vermischen des partikelförmigen Materials mit mindestens einem Bindemittel und gegebenenfalls weiteren Substanzen,
(c) Verarbeiten des in Schritt (b) erhaltenen Gemisches zu einer Paste,
(d) Aufbringen der in Schritt (c) erhaltenen Paste auf einen Träger (1),
(e) Aushärten der auf den Träger (1) aufgebrachten Paste, wobei eine poröse, nicht-quellbare Gerüststruktur ausbildet wird, deren Poren einen Durchmesser von 500 nm bis etwa 5 µm, insbesondere von 1 µm bis etwa 3 µm aufweisen oder/ und die poröse nicht-quellbare Gerüststruktur eine relative Flüssigkeitsaufnahme von etwa 20 Gew.-% bis etwa 50 Gew.-% bevorzugt von etwa 26 Gew.-% bis etwa 44 Gew.-%, bezogen auf das Trockengewicht der porösen, nicht-quellbaren Gerüststruktur, bewirkt und
(f) Anfertigen eines elektrochemischen Sensors, der die in Schritt (e) erhaltene poröse, nicht-quellbare Gerüststruktur als Elektrolytschicht (6), sowie eine Arbeitselektrode (3), eine Gegenelektrode (4) und eine gaspermeable Deckschicht (7) enthält.

11. Verfahren nach Anspruch 10,
**dadurch gekennzeichnet,**
**dass** es weiterhin (i) das Einbringen einer nicht-flüchtige Elektrolytbestandteile enthaltenden Flüssigkeit in die poröse, nicht-quellbare Gerüststruktur und anschließendes Abdampfen der flüchtigen Bestandteile der Flüssigkeit umfasst, oder (ii) das Einbringen einer nicht-flüchtige Elektrolytbestandteile enthaltenden Flüssigkeit in die in Schritt (c) erhaltene Paste umfasst.

12. Verfahren nach Anspruch 11,
**dadurch gekennzeichnet,**
**dass** es weiterhin das Aktivieren des elektrochemischen Sensors umfasst, wobei die Aktivierung durch Inkontaktbringen des elektrochemischen Sensors mit einer Flüssigkeit, insbesondere mit einer wässrigen Flüssigkeit, bewirkt wird, wobei die Flüssigkeit gegebenenfalls einen osmotischen Druck aufweist, welcher dem osmotischen Druck des in der porösen, nicht-quellbaren Gerüststruktur aufgenommenen flüssigen Elektrolyten entspricht.

13. Verfahren zur Bestimmung eines in einem wässrigen Messmedium gelösten gasförmigen Analyten, umfassend die Schritte:
(a) Inkontaktbringen des wässrigen Messmediums mit einem elektrochemischen Sensor nach einem der Ansprüche 1 bis 9, und
(b) Bestimmen des in dem wässrigen Messmedium gelösten gasförmigen Analyten durch Messung eines von dem elektrochemischen Sensor erzeugten Signals.

## Claims

1. Electrochemical sensor for determining a gaseous analyte dissolved in an aqueous measuring medium, comprising:
(a) a working electrode (3) and a counter electrode (4),
(b) a layer of electrolyte (6) located between the working electrode (3) and the counter electrode (4),
(c) a gas permeable covering layer (7), which serves to separate the working electrode (3), the counter electrode (4) and the layer of electrolyte (6) spatially from the aqueous measuring medium,
**characterised in that**
the layer of electrolyte (6) comprises at least a particulate material and at least a binding agent, which together form a porous, non-swellable framework structure, in which the pores of the non-swellable framework structure are provided for absorbing a liquid electrolyte or contain it, and
(i) the pores of the non-swellable framework structure have a diameter from about 500 nm to about 5 µm, particularly from about 1 µm to about 3 µm, and/or
(ii) the porous, non-swellable framework structure brings about a relative liquid absorption from about 20% by weight until about 50% by weight, preferably from about 26% by weight until about 44% by weight, with reference to the dry weight of the porous, non-swellable framework structure.

2. Electrochemical sensor according to claim 1,
**characterised in that**
the particulate material (i) is an inorganic particulate material selected from the group consisting of Controlled Porous Glass (CPG), silica gels and silicates and/or (ii) is a naturally occurring or synthetic aluminosilicate, particularly a synthetic aluminosilicate, and/or (iii) a zeolite, particularly faujasite.

3. Electrochemical sensor according to claim 1 or 2,
**characterised in that**
the particulate material has channels, particularly channels with a diameter of between about 0.1 nm to about 10 nm, and ion exchanger groups as an option.

4. Electrochemical sensor according to one of claims 1 to 3,
**characterised in that**
the particles of the particulate material have an average particle diameter from about 0.1 µm to about 10 µm, particularly from about 1.0 µm to about 5.0 µm.

5. Electrochemical sensor according to one of claims 1 to 4,
**characterised in that**
the binding agent is an organic binding agent, in which the organic binding agent comprises a crosslinked or non-crosslinked synthetic polymer, if necessary, and the crosslinked or non-crosslinked synthetic polymer is a compound selected from the group consisting of a phenol resin, an epoxy resin, a vinyl resin, a PVC copolymer, a two-component epoxy resin, a polyurethane resin system and a UV-hardenable polyacrylate monomer mixture, particularly a phenol resin.

6. Electrochemical sensor according to one of claims 1 to 5,
**characterised in that**
the layer of electrolyte (6) has a thickness from about 5 µm to about 30 µm, particularly from about 10 µm to about 20 µm.

7. Electrochemical sensor according to one of claims 1 to 6,
**characterised in that**
the liquid electrolyte comprises (i) an alkali metal chloride and a pH buffer system, and (ii) a water-soluble polymer, particularly polyethylene glycol and/or polyvinyl pyrrolidone, or an enzyme, particularly carbonic anhydrase, if necessary.

8. Electrochemical sensor according to one of claims 1 to 7,
**characterised in that**
the layer of electrolyte (6) comprises the non-volatile components of the liquid electrolyte before the sensor is put into operation.

9. Electrochemical sensor according to one of claims 1 to 8,
**characterised in that**
the gas permeable covering layer (i) is impermeable for ions and non-volatile components of the aqueous measuring medium and/or (ii) comprises a siloxane, a polysulphone and/or polytetrafluoroethylene and/or (iii) has a thickness from about 5.0 µm to about 30 µm, preferably from about 8.0 µm to about 15 µm.

10. Method for producing an electrochemical sensor according to one of claims 1 to 9, comprising the steps:
(a) preparing at least one particulate material,
(b) mixing the particulate material with at least one binding agent and further substances, if necessary,
(c) processing the mixture obtained in step (b) into a paste,
(d) applying the paste obtained in step (c) to a carrier (1),
(e) hardening the paste applied to the carrier (1), in which a porous, non-swellable framework structure is formed, the pores of which have a diameter from 500 nm to about 5 µm, particularly from 1 µm to about 3 µm and/or the porous, non-swellable framework structure brings about a relative liquid absorption from about 20% by weight to about 50% by weight, preferably from about 26% by weight to about 44% by weight, with reference to the dry weight of the porous, non-swellable framework structure and
(f) producing an electrochemical sensor, which contains the porous, non-swellable framework structure obtained in step (e) as a layer of electrolyte (6) as well as a working electrode (3), a counter electrode (4) and a gas permeable covering layer (7).

11. Method according to
claim 10, **characterised**
**in that**
it also comprises (i) introducing a liquid containing non-volatile electrolyte components into the porous, non-swellable framework structure and then evaporating the volatile components of the liquid or (ii) introducing a liquid containing non-volatile electrolyte components into the paste obtained in step (c).

12. Method according to
claim 11, **characterised**
**in that**
it also comprises activating the electrochemical sensor, in which activating is brought about by bringing the electrochemical sensor into contact with a liquid, particularly with an aqueous liquid, in which the liquid has an osmotic pressure, if necessary, which corresponds to the osmotic pressure of the liquid electrolyte absorbed in the porous, non-swellable framework structure.

13. Method for determining a gaseous analyte dissolved in an aqueous measuring medium, comprising the steps:
(a) bringing the aqueous measuring medium into contact with an electrochemical sensor according to one of claims 1 to 9 and
(b) determining the gaseous analyte dissolved in the aqueous measuring medium by measuring a signal produced by the electrochemical sensor.

## Revendications

1. Capteur électrochimique pour la détermination d'un analyte gazeux dissous dans un milieu de mesure aqueux, comprenant :
(a) une électrode de travail (3) et une contre-électrode (4),
(b) une couche électrolytique (6) située entre l'électrode de travail (3) et la contre-électrode (4),
(c) un couche protectrice (7) perméable aux gaz, qui sert à la séparation dans l'espace de l'électrode de travail (3), de la contre-électrode (4) et de la couche électrolytique (6) par rapport au milieu de mesure aqueux,
**caractérisé en ce que** la couche électrolytique (6) comprend au moins un matériau particulaire et au moins un liant qui forment ensemble une structure de squelette poreuse, non gonflable, les pores de la structure de squelette non gonflable étant destinés à recevoir un électrolyte liquide ou contenant celui-ci et
(i) les pores de la structure de squelette non gonflable présentent un diamètre d'environ 500 nm à environ 5 µm, en particulier d'environ 1 µm à environ 3 µm et/ou
(ii) la structure de squelette poreuse, non gonflable, provoque une absorption relative de liquide d'environ 20% en poids à environ 50% en poids, de préférence d'environ 26% en poids à environ 44% en poids, par rapport au poids sec de la structure de squelette poreuse, non gonflable.

2. Capteur électrochimique selon la revendication 1, **caractérisé en ce que** le matériau particulaire est (i) un matériau particulaire inorganique choisi dans le groupe formé par le verre à porosité contrôlée (Controlled Porous Glass - CPG), les gels siliciques et les silicates et/ou (ii) un aluminosilicate naturel ou synthétique, en particulier un aluminosilicate synthétique et/ou (iii) une zéolithe, en particulier la faujasite.

3. Capteur électrochimique selon la revendication 1 ou 2, **caractérisé en ce que** le matériau particulaire présente des canaux, en particulier des canaux présentant un diamètre entre environ 0,1 nm et environ 10 nm, et éventuellement des groupes échangeurs d'ions.

4. Capteur électrochimique selon l'une quelconque des revendications 1 à 3, **caractérisé en ce que** les particules du matériau particulaire présentent un diamètre moyen de particule d'environ 0,1 µm à environ 10 µm, en particulier d'environ 1,0 µm à environ 5,0 µm.

5. Capteur électrochimique selon l'une quelconque des revendications 1 à 4, **caractérisé en ce que** le liant est un liant organique, le liant organique comprenant le cas échéant un polymère synthétique réticulé ou non réticulé et le polymère synthétique réticulé ou non réticulé étant le cas échéant un composé choisi dans le groupe constitué par une résine phénolique, une résine époxyde, une résine de vinyle, un copolymère de PVC, une résine époxyde à deux composants, un système de résine de polyuréthane et un mélange de monomères de polyacrylate durcissable aux UV, en particulier une résine phénolique.

6. Capteur électrochimique selon l'une quelconque des revendications 1 à 5, **caractérisé en ce que** la couche électrolytique (6) présente une épaisseur d'environ 5 µm à environ 30 µm, en particulier d'environ 10 µm à environ 20 µm.

7. Capteur électrochimique selon l'une quelconque des revendications 1 à 6, **caractérisé en ce que** l'électrolyte liquide (i) comprend un chlorure de métal alcalin et un système tampon du pH et (ii) le cas échéant un polymère soluble dans l'eau, en particulier le polyéthylèneglycol et/ou la polyvinylpyrrolidone, ou une enzyme, en particulier la carboanhydrase.

8. Capteur électrochimique selon l'une quelconque des revendications 1 à 7, **caractérisé en ce que** la couche électrolytique (6) comprend les constituants non volatils de l'électrolyte liquide avant la mise en service du capteur.

9. Capteur électrochimique selon l'une quelconque des revendications 1 à 8, **caractérisé en ce que** la couche protectrice perméable aux gaz (i) est imperméable aux ions et aux constituants non volatils du milieu de mesure aqueux et/ou (ii) comprend un siloxane, une polysulfone et/ou un polytétrafluoroéthylène et/ou (iii) présente une épaisseur d'environ 5,0 µm à environ 30 µm, de préférence d'environ 8,0 µm à environ 15 µm.

10. Procédé pour la fabrication d'un capteur électrochimique selon l'une quelconque des revendications 1 à 9, comprenant les étapes :
(a) préparation d'au moins un matériau particulaire,
(b) mélange du matériau particulaire avec au moins un liant et le cas échéant d'autres substances,
(c) transformation du mélange obtenu dans l'étape (b) en une pâte,
(d) application de la pâte obtenue dans l'étape (c) sur un support (1),
(e) durcissement de la pâte appliquée sur le support (1), une structure de squelette poreuse, non gonflable étant formée, dont les pores présentent un diamètre de 500 nm à environ 5 µm, en particulier de 1 µm à environ 3 µm et/ou la structure de squelette poreuse, non gonflable, provoquant une absorption relative de liquide d'environ 20% en poids à environ 50% en poids, de préférence d'environ 26% en poids à environ 44% en poids, par rapport au poids sec de la structure de squelette poreuse, non gonflable et
(f) fabrication d'un capteur électrochimique, qui contient la structure de squelette poreuse, non gonflable, obtenue dans l'étape (e) comme couche électrolytique (6), ainsi qu'une électrode de travail (3), une contre-électrode (4) et une couche protectrice (7) perméable aux gaz.

11. Procédé selon la revendication 10, **caractérisé en ce qu'**il comprend en outre (i) l'introduction d'un liquide contenant des constituants non volatils de l'électrolyte dans la structure de squelette poreuse, non gonflable et l'évaporation consécutive des constituants volatils du liquide ou (ii) l'introduction d'un liquide contenant des constituants non volatils de l'électrolyte dans la pâte obtenue dans l'étape (c).

12. Procédé selon la revendication 11, **caractérisé en ce qu'**il comprend en outre l'activation du capteur électrochimique, l'activation étant provoquée par la mise en contact du capteur électrochimique avec un liquide, en particulier avec un liquide aqueux, le liquide présentant le cas échéant une pression osmotique qui correspond à la pression osmotique de l'électrolyte liquide absorbé dans la structure de squelette poreuse, non gonflable.

13. Procédé pour la détermination d'un analyte gazeux dissous dans un milieu de mesure aqueux, comprenant les étapes :
(a) mise en contact du milieu de mesure aqueux avec un capteur électrochimique selon l'une quelconque des revendications 1 à 9 et
(b) détermination de l'analyte gazeux dissous dans le milieu de mesure aqueux par mesure d'un signal généré par le capteur électrochimique.
